# FASCICULE DE BREVET EUROPEEN

(11) **EP 1 059 907 B1**
(45) Date de publication et mention de la délivrance du brevet: **15.10.2003**
(21) Numéro de dépôt: 99906315.9
(22) Date de dépôt: 02.03.1999
(51) Int. Cl.: A61F 13/08, A47G 25/90

(54) **DISPOSITIF POUR ENFILER SUR UN MEMBRE UNE ORTHESE COMPRESSIVE TUBULAIRE TELLE QUE BAS, COLLANT OU CHAUSSETTE EN MATERIAU TEXTILE ELASTIQUE TRICOTE**
VORRICHTUNG ZUM ANZIEHEN EINER KOMPRESSIONSORTHESE IN FORM VON AUS ELASTISCHEM TEXTILMATERIAL BESTEHENDEN STÜTZSTRUMPFHOSE, -KNIESTRUMPF, -SOCKEN
DEVICE FOR PULLING ON A LIMB A TUBULAR COMPRESSIVE ORTHOTIC DEVICE SUCH AS A STOCKING, TIGHTS OR SOCK MADE OF KNITTED ELASTIC TEXTILE MATERIAL

(30) Priorité: 02.03.1998 FR 9802487
(43) Date de publication de la demande: 20.12.2000
(73) Titulaire: Innothera Topic International (Societe Anonyme), 94110 Arcueil (FR)
(72) Inventeur: GARDON-MOLLARD, Christian, F-63400 Chamalières (FR)
(74) Mandataire: Dupuis-Latour, Dominique
(86) Numéro de dépôt international: FR9900454
(87) Numéro de publication internationale: WO99044558

(56) Documents cités:
- EP-B- 0 497 858
- FR-A- 2 340 708
- FR-A- 2 647 008
- GB-A- 2 173 390
- SE-B- 447 539
- US-A- 5 031 806

## Description

L'invention a trait au domaine des orthèses compressives tubulaires réalisées en matériau textile élastique tricoté, c'est-à-dire du type "bas de contention".

Ces orthèses, au sens de la présente description, peuvent prendre plusieurs formes. Par exemple, s'agissant des orthèses compressives d'un ou des deux membres inférieurs, il peut s'agir de bas au sens strict (couvrant la cuisse et le jarret), de collants (couvrant les deux membres inférieurs et l'abdomen jusqu'à la ceinture, en une seule pièce), de mono-collants (collants munis d'une seule jambe, destinés à la contention d'un seul des membres inférieurs) ou encore de chaussettes (couvrant le jarret seul).

L'invention s'applique également aux orthèses compressives destinées aux membres supérieurs.

Elle n'est donc pas limitée à un article particulier, mais concerne aussi bien toutes les orthèses compressives tubulaires (c'est-à-dire à l'exception des bandages) telles que celles décrites ci-dessus.

Pour permettre une compression forte du ou des membres, ces orthèses sont réalisées en un matériau élastique, typiquement une maille tricotée de texture très serrée, ce qui entraîne plusieurs séries de difficultés.

L'une de ces difficultés est celle de l'enfilage, en particulier pour les orthèses de type bas ou collant au niveau du pied et de la cheville (là où l'enfilage est le plus difficile et où la compression est la plus forte), avec un risque de mauvais placement, notamment à l'endroit du cou de pied et du talon, qui sont des zones où l'enfilage est toujours assez délicat, et ce d'autant plus que la pression de l'orthèse augmente.

Cette difficulté d'enfilage est généralement accrue par le fait que ces orthèses sont prescrites pour le traitement de pathologies circulatoires qui affectent souvent des patients âgés, malhabiles, parfois handicapés sur le plan moteur, touchées par des phénomènes arthrosiques déformant les mains et les pieds, etc., c'est-à-dire dont la mobilité est en règle générale limitée.

De plus, dans les indications post-chirurgicales, après chirurgie vasculaire veineuse, l'enfilage peut être entravé par les pansements se trouvant sur la jambe après l'acte chirurgical, souvent des pansements de type "pansement américain", très absorbants donc épais, qui gênent l'enfilage mais qu'il est essentiel de ne pas déplacer. L'enfilage peut également être rendu difficile à cause de l'anesthésie générale du patient, qui ne peut contracter volontairement ses muscles pour résister activement au moment de l'enfilage ; il convient dans cette situation de ne pas forcer les articulations passives de l'opéré.

Cette difficulté d'enfilage des orthèses de contention est un obstacle connu des patients et des soignants, et il a été proposé de nombreux types de dispositifs d'enfilage, généralement pour des bas, qui sont pour la plupart des structures métalliques et/ou rigides de manipulation complexe.

Les FR-A-2 340 708 et EP-A-0 497 858 décrivent un tel dispositif, en forme de babouche découpée à l'endroit des orteils ; cet accessoire permet certes de passer plus aisément le bas sur le pied, mais il n'améliore en aucune façon l'enfilage sur le reste du membre, opération qui reste très délicate dans le cas d'un membre portant des pansements, immobilisé et sous anesthésie.

L'un des buts de l'invention est de proposer un dispositif, qui soi d'utilisation simple, pour enfiler sur un membre une orthèse du type précité.

On verra également que le dispositif enfileur de l'invention est peu coûteux à réaliser, ce qui permet de proposer un produit jetable, à usage unique, particulièrement avantageux dans le cas d'orthèses destinées au traitement de l'ulcère veineux car l'enfileur, qui peut se trouver en contact avec les plaies et les sérosités, pourrait être le vecteur de germes s'il était employé chez des patients différents. On verra également que, dans cette dernière indication, l'enfileur à usage unique peut être intégré à l'orthèse pour en faciliter l'enfilage, et détachable de celle-ci ensuite, permettant ainsi au soignant de disposer d'un produit d'une seule pièce, directement enfilable, et dont l'élément servant à l'enfilage peut être dissocié et jeté après utilisation.

Plus précisément, le dispositif de l'invention est caractérisé par un manchon souple en un matériau présentant un faible coefficient de frottement et une forte résistance à la traction, et dimensionné de manière à permettre l'enveloppement du membre sur une longueur correspondant au moins à la longueur de l'orthèse, ce manchon étant apte et destiné à permettre l'enfilage de l'orthèse sur le membre par : a) enveloppement du membre, sur une longueur correspondant au moins à la longueur de l'orthèse, par un manchon souple en un matériau présentant un faible coefficient de frottement et une forte résistance à la traction et à la déchirure ; b) enfilage et mise en place de l'orthèse sur la partie de membre enveloppée par le manchon, cet enfilage étant réalisé manuellement en faisant glisser sur toute sa longueur l'orthèse sur le manchon interposé entre orthèse et membre; et c) une fois l'orthèse enfilée et mise en place, extraction par traction du manchon interposé, ce dernier glissant entre l'orthèse et le membre, qui viennent en contact mutuel au fur et à mesure de l'extraction.

Si l'orthèse est ouverte à ses deux extrémités distale et proximale, l'extraction du manchon est opérée via l'ouverture distale par traction vers l'extérieur dans la région de cette ouverture. Si l'orthèse est fermée à son extrémité distale et ouverte à son extrémité proximale, l'extraction du manchon est opérée via l'ouverture proximale par traction vers l'extérieur dans la région de cette ouverture, puis retrait par passage du manchon ainsi extrait autour du membre, par dessus l'orthèse. >

Ce manchon souple peut être en tissu, notamment un tissu enduit d'un matériau présentant un faible coefficient de frottement. II est avantageusement tubulaire et ouvert à au moins l'une de ses extrémités. Il peut éventuellement comporter deux épaisseurs de matériau retournées l'une sur l'autre par invagination.

Dans une forme de réalisation particulière, particulièrement adaptée à un emploi unique, à l'une de ses extrémités le manchon est solidarisé à l'extrémité distale de l'orthèse par une liaison dissociable.

Pour en faciliter l'extraction, le manchon peut être pourvu à l'une de ses extrémités d'au moins un élément renforcé formant sangle ou poignée de préhension et de traction.

D'autres caractéristiques et avantages de l'invention apparaîtront à la lecture de la description de diverses formes de mise en oeuvre ci-dessous, en référence aux dessins annexés.

Les figures 1, 2 et 3 illustrent trois formes de réalisation possibles de l'enfileur de l'invention.

Les figures 4 et 5 illustrent, selon deux variantes possibles, un enfileur selon l'invention associé à une orthèse destinée plus particulièrement au traitement des pathologies de l'ulcère veineux.

Les figures 6 et 7 illustrent les étapes successives de la mise en oeuvre de l'enfileur de l'invention, respectivement avec retrait de l'enfileur par le haut ou par le bas.

L'enfileur de l'invention est constitué d'un manchon souple, déformable, formé dans cet exemple d'une pièce de tissu susceptible d'entourer de façon enveloppante le membre (jambe ou bras) devant recevoir l'orthèse, en le couvrant de tous côtés.

Ce manchon peut être réalisé par exemple à partir d'une simple pièce de tissu enroulée autour du membre, mais il est de préférence réalisé sous forme d'un article tubulaire, c'est-à-dire que l'un des côtés de la pièce de tissu est cousu avec un côté opposé de manière à définir par un contour fermé une ouverture du manchon dans laquelle sera introduit le membre. Cette ouverture, qui correspond après enfilage du manchon à l'extrémité proximale du membre, sera désignée "extrémité supérieure" par la suite.

Le manchon est ainsi ouvert à son extrémité supérieure ; il peut être ouvert aussi à son extrémité inférieure pour laisser éventuellement passer l'extrémité distale du membre ou, au contraire, fermé à cette extrémité inférieure, le manchon ayant alors une forme de sac ou de poche. On comprendra par la suite, lorsque l'on exposera la mise en oeuvre de l'enfileur de l'invention, qu'un manchon à deux extrémités ouvertes (variante illustrée sur les diverses figures) est applicable à l'enfilage de tous types d'orthèses, tandis qu'un manchon à une seule extrémité ouverte n'est applicable qu'à l'enfilage d'orthèses de type "pied ouvert".

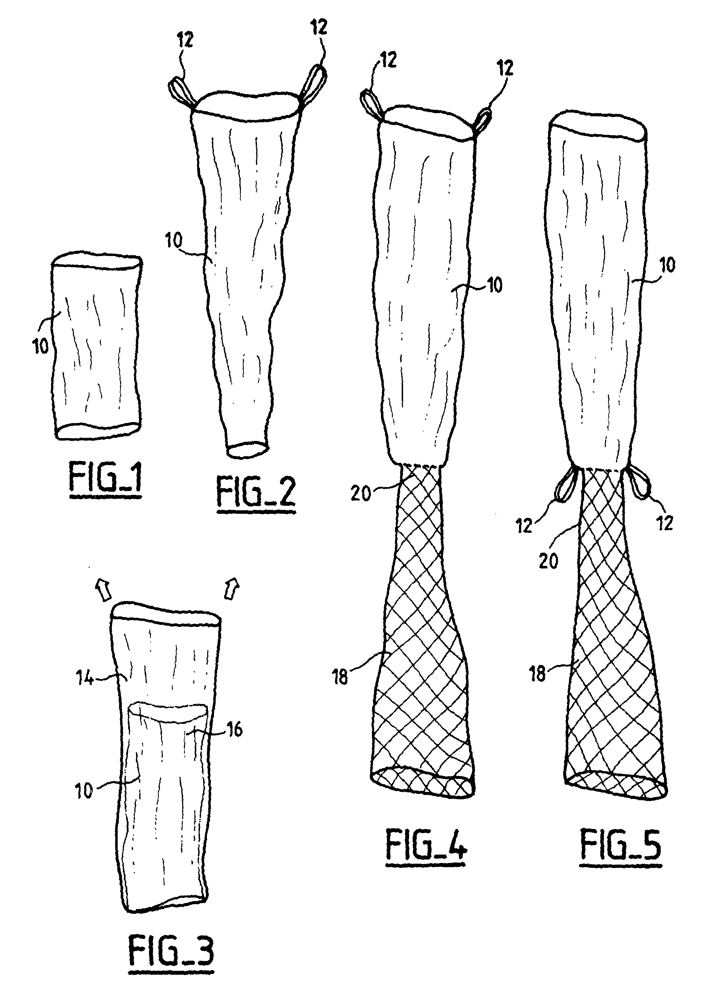

La forme du manchon, référencé 10 sur les figures, peut être approximativement cylindrique, comme illustré sur la figure 1 (pour un enfileur de bas et chaussettes, par exemple) ou légèrement conique et allongée (pour un enfileur de bas ou d'orthèses remontant au-dessus du genou), comme illustré sur la figure 2.

Il est avantageusement prévu un accessoire de préhension et de traction 12 en forme de sangle, poignet, dragonne, anse, etc. pour aider le patient ou le soignant à retirer plus aisément l'enfileur après mise en place de l'orthèse (voir plus bas).

L'enfileur est soit simple, c'est-à-dire constitué d'une seule épaisseur de tissu, soit double comme illustré figure 3, c'est-à-dire avec deux épaisseurs 14, 16 par repli d'une épaisseur de tissu sur elle-même en "doigt de gant" par invagination, ce qui facilitera encore plus l'enfilage de l'orthèse compressive et le retrait de l'enfileur.

Le matériau du manchon est un matériau présentant un faible coefficient de frottement et une forte résistance à la rupture (traction et déchirure).

Les notions de "faible coefficient de frottement" et de "forte résistance à la traction et à la déchirure" sont relatives, et signifient que le procédé tel qu'il sera décrit ci-dessous devra pouvoir être mis en oeuvre de façon satisfaisante à la main sans l'aide d'aucun accessoire supplémentaire, sans déplacement de l'orthèse une fois positionnée ni déchirure de l'enfileur pendant son extraction.

Les propriétés mécaniques requises à cet effet pourront varier en fonction du caractère plus ou moins compressif de la maille de l'orthèse (les classes de compression III ou IV imposant une plus grande force de traction, donc un matériau plus résistant), de la structure de cette maille et du matériau de l'orthèse, certaines caractéristiques (guipage, incorporation d'élasthanne ou d'élasto-diène par exemple) pouvant avoir une incidence sur les coefficients de frottement de l'orthèse par rapport à la peau et par rapport à l'enfileur.

Un matériau qui convient particulièrement est par exemple le *Stabilkote 4*, qui est un tissu, utilisé notamment en voilerie de marine. Ce matériau est réalisé par tissage de fils de chaîne et de trame en polyamide nylon 6.6 de 30 deniers chacun, imprégnation de résine mélamine et enduction de polyuréthanne. Ce tissu présente un poids spécifique de 42 ± 2 g/m² ; il est donc très léger et néanmoins extrêmement résistant à la déchirure ; son enduction de polyuréthanne lui confère un très faible coefficient de frottement aussi bien par rapport à la peau que par rapport à la surface de pansements ou bandes, par rapport à la maille tricotée d'une orthèse de compression ou par rapport à lui-même.

Comme on le comprendra à la lecture de la présente description, la simplicité de conception et d'emploi du manchon souple de l'invention résulte notamment du glissement du tissu sur lui-même, c'est-à-dire que le tube doit être invaginé sur une certaine longueur avant d'être posé sur la jambe qui va recevoir l'orthèse. Cette manipulation peut d'ailleurs être simplifiée pour l'utilisateur par un système de couleurs différentes (le rouge devant être rentré dans le bleu par exemple).

La taille de l'enfileur doit être de diamètre suffisant pour permettre l'enfilage sur le membre, typiquement une jambe, qui peut, dans le cas d'une pathologie d'ulcère veineux, présenter une ulcération simple ou multiple sur laquelle on aura disposé pour les soins et pour le pansement des compresses ou des pansements américains. Cette dimension peut être d'au moins 25 à 30 cm de diamètre minimal pour pallier tous les cas de figure habituels, si l'enfileur a une forme cylindrique ; dans le cas d'une forme conique, les dimensions seront adaptées aux différentes anatomies de mollets et de cuisses si l'enfileur doit remonter au-dessus du genou.

Il n'est pas gênant de surdimensionner l'enfileur, notamment pour tenir compte de l'épaisseur des pansements éventuellement présents sur le membre. Ce surdimensionnement entraînera la formation de plis sur l'enfileur au moment de la mise en place de l'orthèse, mais, comme on le comprendra lorsque l'on exposera en détail le procédé d'enfilage, ces plis ne sont pas gênants, bien au contraire, car le coefficient de frottement du matériau de l'enfileur sur lui-même est très faible, ce qui facilitera encore l'extraction de ce dernier.

En particulier, le surdimensionnement peut permettre une invagination plus aisée de l'extrémité distale de l'enfileur, par exemple le tiers inférieur (distal) sera invaginé dans le tiers médian, le tiers supérieur (proximal) ne servant essentiellement qu'à exercer la traction pour enlever l'ensemble de l'enfileur.

Dans certaines pathologies, telles que les ulcérations de jambes, en général sièges d'infections et de lésions trophiques importantes, l'enfileur devra être à usage unique, c'est-à-dire jetable. Compte tenu de son très faible coût, tant de matière que de fabrication, un tel usage unique est tout à fait réaliste.

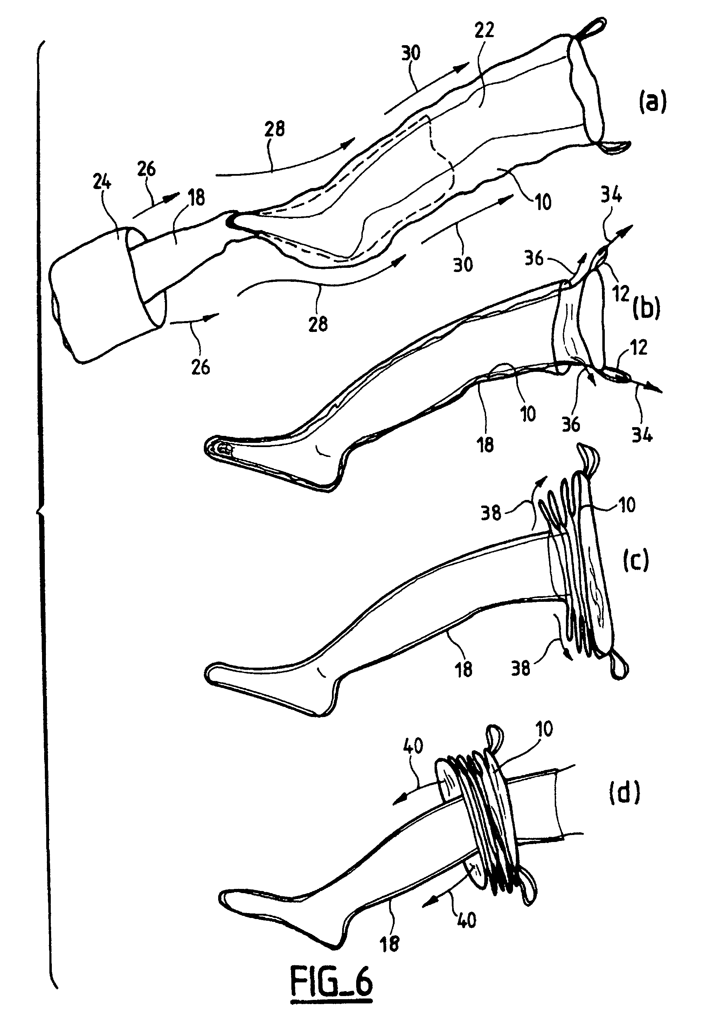

Dans ce cas, l'enfileur peut être avantageusement solidarisé à l'orthèse, comme illustré sur les figures 4 et 5. Sur ces figures, la référence 18 désigne une orthèse, notamment une orthèse jetable pour la contention de la jambe à la suite d'un ulcère veineux, telle que décrite dans le WO-A-97/47262 (Innothéra Topic International), auquel on pourra se référer pour de plus amples détails. L'enfileur attenant 10, simple ou double, est cousu en 20 à l'extrémité inférieure (extrémité distale, côté pied) de l'orthèse 18 par un point de surfilage large ou avec un fil sécable, à une distance suffisante de la pointe du pied, afin de permettre après enfilage de l'orthèse de détacher l'enfileur par simple coupure de la maille. D'autres modes de solidarisation, par exemple par soudure haute fréquence, sont bien entendu envisageables dès lors qu'ils permettent aisément la désolidarisation de l'orthèse et de l'enfileur après emploi.

Par ailleurs, pour un article universel utilisable avec tous types d'orthèses compressives (bas-cuisse, chaussettes, collants), avec pieds ouverts ou bien pieds fermés, l'enfileur pourra être rendu "ouvrable" sur un de ses côtés par des moyens divers tels que bandes agrippantes, colle, boutons-pression, laçage, fermeture à glissière, etc.

Diverses variantes de réalisation de l'enfileur sont possibles, selon que l'on souhaite retirer l'enfileur par le haut (les anses 12 seront alors situées côté proximal, comme sur la figure 4) ou vers le bas (les anses étant alors situées côté distal, comme sur la figure 5).

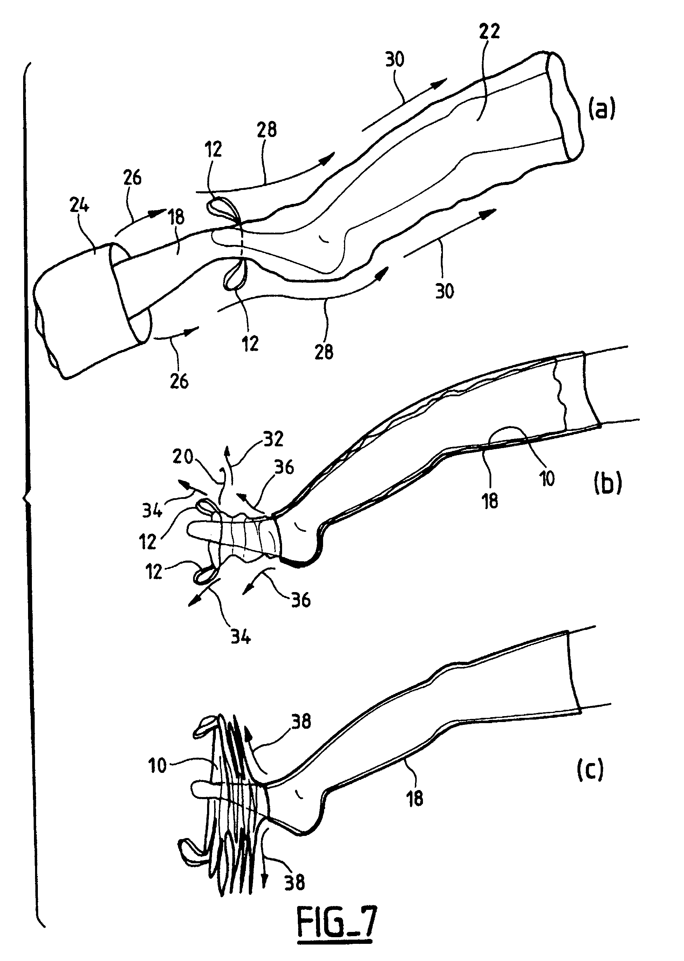

On va maintenant décrire en détail les diverses étapes du procédé d'enfilage en référence aux figures 6 et 7 qui en exposent deux variantes possibles :
― la figure 6 vise le cas où l'enfileur, qui est du type illustré figure 2, est extrait par le haut (côté proximal), comme cela doit être le cas avec une orthèse telle qu'illustrée, en forme de bas fermé au niveau des orteils (cet exemple n'est cependant pas limitatif et le procédé s'applique aussi bien à l'enfilage de tous types de bas, chaussettes, collants, etc., ouverts ou non au niveau du pied ou de la cheville) ; dans cette variante l'enfileur est très avantageusement invaginé au préalable, comme expliqué plus haut, par exemple avec le tiers distal invaginé dans le tiers médian ;
― la figure 7 illustre le cas où l'enfileur est retiré par le bas (côté proximal), ce qui implique que l'orthèse soit ouverte à son extrémité distale, au niveau du pied ou de la cheville ; cette figure 7 vise le cas de l'enfilage d'une orthèse telle qu'illustrée figures 5 avec un enfileur attenant, mais cet exemple n'est pas limitatif et le procédé s'applique aussi bien à l'enfilage de tout type d'orthèse ouverte au niveau du pied ou de la cheville, avec ou sans enfileur attenant.

La première étape, illustrée en a figures 6 et 7, consiste à mettre en place l'enfileur 10 sur le membre 22, le cas échéant avec ses pansements. Dans le cas de la figure 7, où l'orthèse 18 est attenante à l'enfileur, celle-ci est retournée initialement à l'envers, c'est-à-dire avec sa face interne (celle destinée à venir en contact avec le membre) tournée vers l'extérieur.

L'orthèse est ensuite retournée progressivement à l'endroit (comme en 24 sur la figure 7)) et glissée sur la jambe (flèches 26, 28, 30), ce glissement étant facilité par le très faible coefficient de frottement du matériau de l'enfileur 22, de sorte que l'enfilage est réalisé très aisément à deux mains par le patient ou le soignant, sans l'aide d'aucun accessoire particulier ni assistance d'une tierce personne.

On aboutit alors à la situation illustrée en b figures 6 et 7, avec l'orthèse 18 mise en place sur la jambe, l'enfileur 10 étant interposé entre jambe et orthèse.

Dans le cas de l'orthèse avec enfileur attenant de la figure 7, il est nécessaire de couper ou retirer alors le fil 20 de liaison de l'orthèse à l'enfileur (flèche 32) pour séparer ces deux éléments.

L'étape suivante, illustrée en b et c sur les figures 6 et 7, consiste à extraire l'enfileur 10 par traction sur l'une de ses extrémités, soit son extrémité proximale (extraction par le haut, figure 6, cette extraction étant grandement facilitée par l'invagination de l'enfileur) soit son extrémité distale si cela est possible (extraction par le bas, figure 7) ; cette traction peut être notamment exercée sur les anses 12 (flèches 34).

Compte tenu de son très faible coefficient de frottement par rapport à la peau, d'une part, et par rapport au tissu de l'orthèse, d'autre part, il sera aisé d'extraire ainsi complètement l'enfileur (flèches 36, 38) . En revanche, l'orthèse 18, du fait de son coefficient de frottement élevé par rapport à la peau, et du fait de l'effort de striction dû à l'élasticité de la maille, ne se déplacera pas par rapport aux membres pendant cette opération d'extraction de l'enfileur et conservera donc le positionnement qui lui a été donné à la mise en place.

Dans le cas de la figure 6, où l'on a extrait l'enfileur par le haut, l'enfileur est ensuite retiré de la jambe en le passant par dessus l'orthèse 18 comme illustré en d (flèche 40) ; bien entendu, pour permettre cette opération, l'enfileur doit dans ce cas être, d'une part, ouvert à ses deux extrémités et, d'autre part, de diamètre suffisant pour pouvoir être enlevé par le haut de la cuisse.

## Revendications

1. Un dispositif pour enfiler sur un membre (22) une orthèse compressive tubulaire (18) telle que bas, collant ou chaussette en matériau textile élastique tricoté, le cas échéant avec des pansements ou bandages présents sur le membre,
dispositif **caractérisé en ce qu'**il est formé d'un manchon souple (10) en un matériau présentant un faible coefficient de frottement et une forte résistance à la traction,
**en ce que** ce manchon est dimensionné de manière à permettre l'enveloppement du membre sur une longueur correspondant au moins à la longueur de l'orthése,
et **en ce qu'**il est apte et destiné à permettre l'enfilage de l'orthèse sur le membre par :
a) enveloppement du membre, sur une longueur correspondant au moins à la longueur de l'orthèse, par ledit manchon souple (10),
b) enfilage et mise en place de l'orthèse sur la partie de membre enveloppée par le manchon, cet enfilage étant réalisé manuellement en faisant glisser sur toute sa longueur l'orthèse sur le manchon interposé entre orthèse et membre, et
c) une fois l'orthèse enfilée et mise en place, extraction par traction du manchon interposé, ce dernier glissant entre l'orthèse et le membre, qui viennent en contact mutuel au fur et à mesure de l'extraction.

2. Le dispositif de la revendication 1, **caractérisé en ce que** le manchon souple est un manchon en tissu, notamment en un tissu enduit d'un matériau présentant un faible coefficient de frottement.

3. Le dispositif de la revendication 1, **caractérisé en ce que** le manchon souple est tubulaire et ouvert à au moins l'une de ses extrémités.

4. Le dispositif de la revendication 1, **caractérisé en ce que** le manchon souple comporte deux épaisseurs de matériau (14, 16) retournées l'une sur l'autre par invagination.

5. Le dispositif de la revendication 1 **caractérisé en ce que**, à l'une de ses extrémités, le manchon souple est solidarisé à l'extrémité distale de l'orthèse par une liaison dissociable (20).

6. Le dispositif de la revendication 1, **caractérisé en ce que** le manchon souple est pourvu à l'une de ses extrémités d'au moins un élément renforcé formant sangle ou poignée (12) de préhension et de traction.

7. Le dispositif de la revendication 1, **caractérisé en ce qu'**il est apte et destiné à permettre l'enfilage sur le membre d'une orthèse ouverte à ses deux extrémités distale et proximale, par extraction du manchon via l'ouverture distale par traction vers l'extérieur dans la région de cette ouverture (figure 7).

8. Le dispositif de la revendication 1, **caractérisé en ce qu'**il est apte et destiné à permettre l'enfilage sur le membre d'une orthèse fermée à son extrémité distale et ouverte à son extrémité proximale, par extraction du manchon via l'ouverture proximale par traction vers l'extérieur dans la région de cette ouverture, puis retrait par passage du manchon ainsi extrait autour du membre, par dessus l'orthèse (figure 6).

## Patentansprüche

1. Vorrichtung zum Überstreifen einer kompressiven, röhrenförmigen Orthese (18), wie z.B. ein Strumpf, eine Strumpfhose oder ein Kniestrumpf aus einem textilen, elastischen, gestrickten Material über ein Körperglied (22), gegebenenfalls mit Verbänden oder Bandagen, die an dem Körperglied vorhanden sind,
wobei die Vorrichtung **dadurch gekennzeichnet ist, dass** sie aus einer nachgiebigen Hülse (10) aus einem Material gebildet ist, welches einen geringen Reibungskoeffizienten und eine starke Widerstandsfähigkeit gegen Zug aufweist,
**dass** die Hülse in einer Weise dimensioniert ist um die Umhüllung des Körperglieds auf einer Länge entsprechend mindestens zu der Länge der Orthese zu erlauben,
und **dass** sie geeignet und bestimmt ist, das Überstreifen der Orthese auf dem Körperglied zu erlauben durch:
a) Umhüllen des Körperglieds über eine Länge entsprechend mindestens zu der Länge der Orthese durch die nachgiebige Hülse (10),
b) Überstreifen und Platzieren der Orthese auf dem Teil des Körperglieds, welches durch die Hülse umhüllt ist, wobei dieses Überstreifen von Hand realisiert ist durch gleiten lassen der Orthese über ihre gesamte Länge auf der Hülse, welche zwischen die Orthese und dem Körperglied zwischengesetzt ist, und
c) wenn die Orthese einmal platziert ist, Entfernen durch Ziehen der zwischengeschalteten Hülse, wobei diese Letztere zwischen der Orthese und dem Körperglied gleitet, welche nach und nach gegenseitig in Kontakt gelangen bei der Entfernung.

2. Vorrichtung nach Anspruch 1, **dadurch gekennzeichnet, dass** die nachgiebige Hülse eine Hülse aus einer Textilware ist, insbesondere ein Gewebe, welches beschichtet ist mit einem Material, das einen niedrigen Reibungskoeffizienten aufweist.

3. Vorrichtung nach Anspruch 1, **dadurch gekennzeichnet, dass** die nachgiebige Hülse röhrenförmig und an mindestens einem ihrer Enden offen ist.

4. Vorrichtung nach Anspruch 1, **dadurch gekennzeichnet, dass** die nachgiebige Hülse zwei Dicken eines Materials (14, 16) aufweist, die übereinander umgeschlagen sind durch Invagination.

5. Vorrichtung nach Anspruch 1, **dadurch gekennzeichnet, dass** an einem ihrer Enden die nachgiebige Hülse mit dem distalen Ende der Orthese durch eine lösbare Verbindung (20) fest verbunden ist.

6. Vorrichtung nach Anspruch l, **dadurch gekennzeichnet, dass** die nachgiebige Hülse an einem ihrer Enden mit mindestens einem verstärkten Element versehen ist, welches einen Gurt oder Griff (12) zum Greifen und Ziehen bildet.

7. Vorrichtung nach Anspruch 1, **dadurch gekennzeichnet, dass** sie geeignet und bestimmt ist, das Überstreifen auf einem Körperglied von einer offenen Orthese an ihren zwei distalen und proximalen Enden zu erlauben durch Entfernen der Hülse über die distale Öffnung durch Ziehen in Richtung nach außen im Bereich dieser Öffnung (Fig. 4).

8. Vorrichtung nach Anspruch 1, **dadurch gekennzeichnet, dass** sie geeignet und bestimmt ist, das Überstreifen auf einem Körperglied einer Orthese zu erlauben, welche an ihrem distalen Ende geschlossen und an ihrem proximalen Ende offen ist, durch Entfernen der Hülse über die proximale Öffnung durch Ziehen in Richtung nach außen im Bereich dieser Öffnung und danach Zurückziehen durch Hindurchführen der Hülse, um solchermaßen entfernt um das Körperglied herum, von oben der Orthese (Fig. 6).

## Claims

1. A device for putting onto a limb (22) a tubular compressive orthosis (18) such as a stocking, tights or a sock, of knitted elastic textile material, optionally with dressings or bandages present on the limb,
said device being **characterized in that** it is made from a flexible sleeve (10) of material that presents a low coefficient of friction and high traction strength,
**in that** said sleeve is dimensioned in such a manner as to enable the limb to be enveloped over a length that corresponds at least to the length of the orthosis,
and **in that** it is adapted and devised to enable putting the orthosis on the limb as follows:
a) the limb is enveloped, over a length corresponding at least to the length of the orthosis, by said flexible sleeve (10);
b) the orthosis is put on and put into place over that portion of the limb which is enveloped by the sleeve, with this being performed manually causing the orthosis to slide over its entire length on the sleeve interposed between the orthosis and the limb; and
c) once the orthosis has been put on and put into place, the interposed sleeve is extracted by traction, the sleeve sliding between the orthosis and the limb which then come mutually into contact as the sleeve is extracted.

2. The device of claim 1, **characterized in that** the flexible sleeve is a sleeve of cloth, in particular of a cloth coated in a substance having a low coefficient of friction.

3. The device of claim 1, **characterized in that** the flexible sleeve is tubular and open at at least one of its ends.

4. The device of claim 1, **characterized in that** the flexible sleeve has two thickness of material (14, 16) turned one inside the other by invagination.

5. The device of claim 1, **characterized in that** the flexible sleeve is secured at one of its ends to the distal end of the orthosis via a releasable link (20).

6. The device of claim 1, **characterized in that** the flexible sleeve is provided at one of its ends with at least one reinforced element constituting a strap or handle (12) for grasping and pulling.

7. The device of claim 1, **characterized in that** it is adapted and devised to enable putting on the limb an orthosis which is open at both its distal and its proximal end, the sleeve being extracted via the distal opening by being pulled outwards in the vicinity of said opening (Figure 7).

8. The device of claim 1, **characterized in that** it is adapted and devised to enable putting on the limb an orthosis which is closed at its distal end and open at its proximal end, the sleeve being extracted via the proximal opening by pulling outwards in the vicinity of said opening, and then withdrawing the sleeve extracted in this way by causing it to pass around the limb, over the orthosis (Figure 6).
